# EUROPEAN PATENT SPECIFICATION

(11) **EP 1 399 102 B1**
(45) Date of publication and mention of the grant of the patent: **10.05.2006**
(21) Application number: 02744050.2
(22) Date of filing: 27.06.2002
(51) Int. Cl.: A61F 5/14, A43B 13/38

(54) **ORTHOPAEDIC APPLIANCE FOR IMPROVED GAIT**
ORTHOPÄDISCHES GERÄT ZUR GANGVERBESSERUNG
APPAREIL ORTHOPEDIQUE POUVANT AMELIORER LA DEMARCHE

(30) Priority: 27.06.2001 SE 0102278
(43) Date of publication of application: 24.03.2004
(73) Proprietor: CAMP SCANDINAVIA AB, 254 67 Helsingborg (SE); Global Orthopaedic Laboratory AB, 254 33 Helsingborg (SE)
(72) Inventor: BENGTSSON, Kenneth, S-286 00 Svalöv (SE); SÖDERBERG, Bengt, S-254 33 Helsingborg (SE)
(74) Representative: Akerman, Marten Lennart
(86) International application number: PCT/SE2002/001276
(87) International publication number: WO 2003/002042

(56) References cited:
- EP-A2- 0 931 470
- GB-A- 1 433 481

## Description

This invention relates generally to orthopaedic appliances such as, e.g. prostheses and orthoses for improving a handicapped or disabled person's gait, and in particular to soles having areas of different flexural resistance.

### Background

Various inlays and appliances for compensating mere anatomical insufficiencies have been known for a long time. Those appliances take into account the statical aspects of weight and force distribution of the foot and lower limb. Attempts to take into account both statical and dynamical forces, i.e., forces generated during a stride, includes the patent publication SE 89967 to Ehrlich 1934. That document discloses an orthopaedic footwear together with method and device for its production.

In EP 0,931,470 A2 to Pavesi is disclosed a sandwich type footwear stiffening element of rigid or at least semi-rigid behaviour, usable as part of a sole unit or insole.

In GB 1,433,481 to Revill is disclosed a shoe insole comprising a non-woven bonded fibre mass having a greater density at a rear portion of the insole than at a forepart portion thereof and the rear portion being more rigid than the forepart portion.

In US 4,085,758 to Castiglia is disclosed a weight-redistribution orthopaedic appliance adapted to be attached to the sole of a shoe. That orthopaedic appliance is a non-resilient pad that is attached to the sole of a shoe forward of a break line of the sole, the pad being sufficiently flexible to be readily contoured to the sole's surface.

### Summary of the invention

The present invention solves the above-mentioned problem of compensating not only for mere anatomical insufficiencies but also for both statical and dynamical forces, i.e., forces generated during a handicapped or disabled person's stride, by providing an orthopaedic appliance in the shape of a sole having areas of different flexural resistance. Boundary zones, or boundary lines defming the boundaries of said areas has been given a certain favourable shape. The sole on its own or as an integral part of a prosthesis or an orthosis, is conceived, constructed and reinforced so as to adapt the position and/or the shape of the so called release line to relieve a patient's problems. The invention is based on the inventors realisation of the importance to control the extension of the so called progression line, particularly when dealing with an amputated foot or an impaired muscle. The invention is an important component in the struggle for preventing injuries affecting the knee, hip and back, among patients with impaired gait, as has been possible to describe with the aid of three dimensional gait analysis.

The sole according to the invention solves the problem of correcting the position and shape of the progression line and the release line by being reinforced laterally and at the same time more frontally than a sole of a normal shoe. The sole supports the foot in a certain manner and the ground reaction-force progression-line is brought back to normal. This guiding and supportive effect makes it easier for the person to hold the foot in a straight position throughout a stride and also to avoid gait deviations at the ankle, knee and hip joints. The patient is spared an unphysiological gait of having to turn the foot at each step. Instead the leg can be swung in a straight line. A more natural gait is achieved. The above mentioned results including the transfer or shift of the ground reaction-force progression-line has been investigated using three-dimensional gait analysis.

The sole can be manufactured in carbon fibre reinforced composite materials or the like and having different number of layers in different parts of the sole, including different types of and number of layers of weave and fabric with crossing fibre directions, of said fibres. The invention is not dependent upon the type of material used.

### Brief description of the drawings

Fig. 1 is a plan view illustrating the progression line of the ground reaction : force under the foot.
Fig. 2 shows a number of alternative release lines.
Fig. 3 shows areas with different flexural resistance.
Fig. 4 shows a top view and side view showing different layers of the sole.

### Detailed description of the preferred embodiments

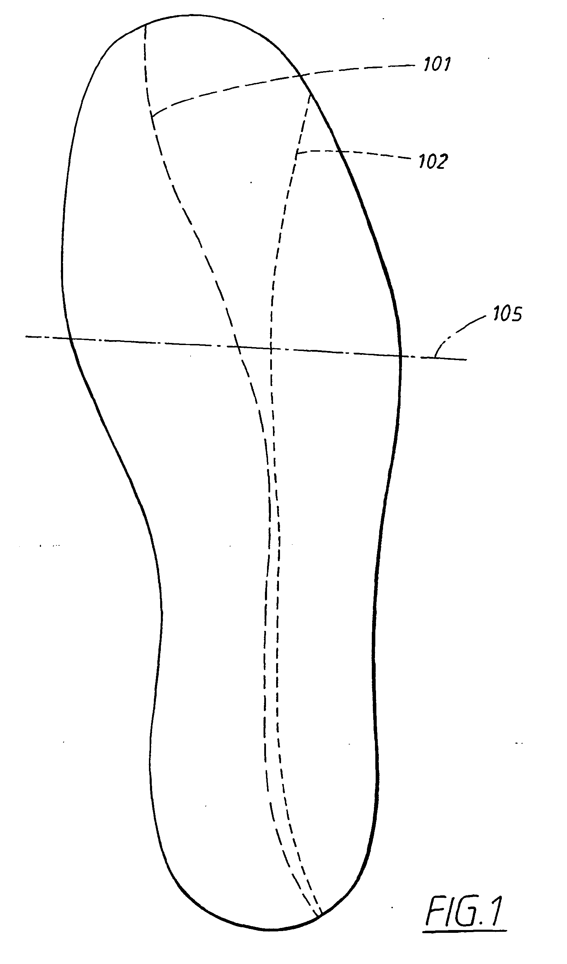

The development of pressure sensitive plates have made it possible for the inventors to measure the reactive force from the floor towards the sole of the foot or towards the sole of footwear. In fig. 1 is shown a sole with both a defective 102 and a normal 101 progression line of the reactive force drawn into the figure for comparison.

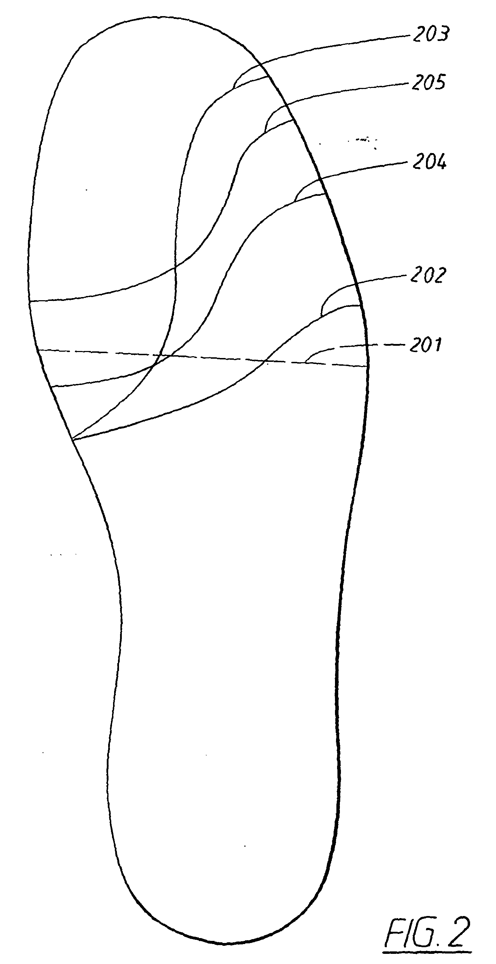

When a person with normal gait walks, the result is a neutral progression line 101. The progression line 101 begins at the posterior part of the heel and advances forward towards the big toe. At the end stage the foot is bent at the metatarsophalangeal joints and the sole is bent along a so-called release line 105. A normal release line 105 is shown in the figure and it runs along the ball of the foot. Good footwear is built to bend along this normal release line 105. During normal gait there is no need to turn the foot at each step and the leg can be swung in a straight line. A number of alternative release lines 202-205, corresponding to different person's pathology in gait, can be seen on the sole in fig. 2. The normal release line 201 is shown as a dashed line.

As an example people suffering from paresis and being partly paralysed or having a partly amputated foot cannot walk properly with normal footwear. For such a person the disability will give rise to a progression line 102 as can be seen in figure 1 . The line 102 deflects towards the little toe because the foot (or what is left of it) cannot withstand the load. Because of the deflected progression line 102, i.e. the ground reactive forces working in a faulty point of action, the foot must be turned in the swing phase and the leg cannot be swung in a straight line. Furthermore the knee will have an increased varus moment and the person will get a limping pattern, which in turn will give rise to problems with the persons' ankle, knee, and hip joints.

It is known how to build for example runner's shoes with soles for preventing supination and pronation. Those shoes are built up mainly around the heel and the metatarsus so as to straighten the foot up and tilt it medially or laterally. This tilt has by no means any influence on the release line, which is arranged, at the same position as in normal shoes.

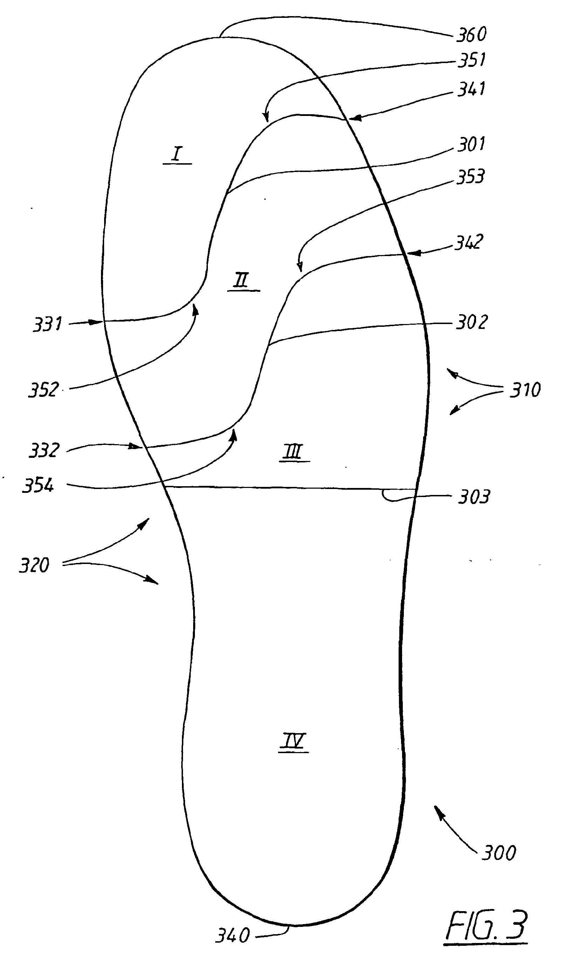

In fig.3 is shown areas of different flexural resistance of a sole according to the invention. The sole is conceived, constructed and reinforced so as to adapt the position and/or the shape of the release line to a patient's problems. The sole is reinforced laterally and frontally compared to a sole of a normal shoe, i.e. the portions of the foot (or what is left of it) that corresponds to the little toe portion of the foot, and the lateral part of the foot are given a stiffer support than is the areas corresponding to the big toe and the medial front part of the foot. The sole supports in this way the foot and thereby the ground counter-force progression-line is brought back to normal. This guiding and supportive effect makes it easier for the person to hold the foot in a straight position throughout the stride, and gait deviations at the ankle, knee, and hip joints are avoided. The patient is spared the inconvenience of having to turn the foot at each step and the leg can be swung in a straight line. A more natural gait is achieved.

Again turning to figure 3, the sole has flexible areas of different flexural resistance. As an example in figure 3, the area labelled I is the most flexible. The area labelled II is a bit less flexible than area I. The area labelled III is a bit less flexible than area II. The area labelled IV is the stiffest area. Another positive side of this construction is a spring effect produced at toe-off, which preserves energy.

A such flexible area of the sole is bounded by either an outer limit or edges of the sole and one or more boundary zones or boundary lines 301, 302, 303 or only by boundary lines. In the figures, boundary lines are drawn as thin lines, but in a real product these lines can have a width of up to a few millimetres incorporating an area of intermediate flexural resistance.

Two boundary lines 301, 302 of a preferred embodiment of the invention as shown in figure 3 begin at a lateral side edge 310 of the sole 300, extend towards a midline of the sole (not shown), extends at the same time in a posterior direction in such a way that when reaching a medial side edge 320 of the sole 300, an end point 331, 332 of said boundary lines 301, 302 is arranged decidedly more posterior, i.e. closer to the heel end 340 of the sole 300, than is a start point of said line. In this way the progression line will be moved in a direction towards the big toe. The release line will at the same time be moved or tilted towards a more normal or pronated position. A third boundary line 303 is arranged to extend in a straight line.

As can be seen in figure 3 the boundary lines can assume curved shapes reminding of an "S" or they can be curved otherwise or be straight. Tests have shown that shapes of the boundary lines resembling an elongated "S" in general, also resembling the hyperbolic tangent function graph, the sigmoid function graph, the integral sign or the like is well suited for achieving proper function. A bend 351, 352 in a boundary line 301, 302 can be directed towards the toe end 360 of the sole or towards the heel end 340. A bend can also be directed towards, or partly towards, the lateral 310 or medial 320 side edge of the sole 300. In a preferred embodiment three boundary lines 301-303 are arranged. The first boundary line 301 is arranged with a first bend 351 being convex in a direction towards the toe end 360 of the sole 300. Said boundary line 301 is also arranged with a second bend 352 being concave in a direction towards the toe end 360 of the sole 300. The second boundary line 302 is arranged having a first bend 353 being convex in a direction towards the toe end 360 of the sole 300. Said second boundary line 302 is also arranged having a second bend 354 being concave in a direction towards the toe end 360 of the sole 300.

The sole can be manufactured of carbon fibre reinforced composite materials or the like. The invention is however not dependent upon the type of material used. Every material with a suitable flexibility can be used, such as glass fibre reinforced plastics, other plastics, wood, steel or other metals. Preferred materials include KEVLAR® (aramid) fibre, carbon fibre, glass fibre, steel and thermoplastic resin.

One preferred way of establishing the areas of different flexural resistance is to manufacture the sole of a number of layers of a material. Referring to figure 3, the sole could be constructed with one layer material in area I, two layer material in area II, three layer material in layer III, and four layer material in layer IV.

In a preferred embodiment, however, the area I is composed of two layers of crossed direction carbon fibre fabric together with two layers of single direction carbon fibre material. Area II is composed of two layers of single direction fibre material together with two layers of crossed fibres together with two layers of single direction fibre material again. Area III is composed of the same as layer II but with an additional layer of single direction fibre material. Area IV is composed of three layers of single direction fibre material together with three layers of crossed fibres together with two layers of single direction fibre material again.

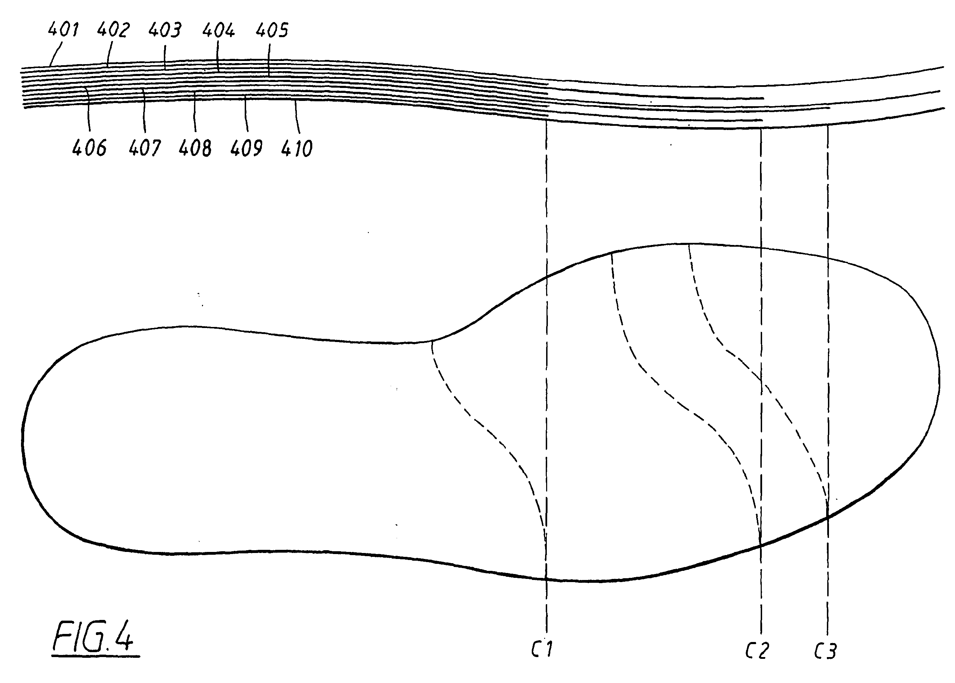

In fig. 4 another embodiment is shown. The sole is shown from above and from the side. The corresponding positions and lines C1, C2, and C3 are marked in both views. Different layers of different extensions and materials are shown. The top layer 401 extends to the full width of the sole and consists of 45 degrees KEVLAR® fabric (45 degrees between warp and weft). The second layer 402 extends to the C1 line and consists of 45 degrees carbon fibre fabric. The third layer 403 extends to the line C2 and consists of straight carbon fibres. The fourth layer extends to line C1 and consists of 45 degrees carbon fibre fabric. The fifth layer 405 extends the full sole and consists of 45 degrees carbon fibre fabric also. The sixth layer extends to the line C3 and consists of straight carbon fibres. The seventh layer extends to C1 and consists of 45 degrees carbon fibre fabric. The eighth layer extends to C2 and consists of straight carbon fibres. The ninth layer extends to C 1 and consists of 45 degrees carbon fibre fabric. The tenth and bottom layer extends the full width of the sole and consists of 45 degrees KEVLAR® fabric.

## Claims

1. A sole (300) to be placed under a patients' foot inside a shoe or being part of an orthopaedic appliance, comprising material arranged to provide said sole with a first flexural resistance at a first area of said sole and a second flexural resistance at a second area of said sole, **characterised in that** said first and second areas are arranged such that the little toe and lateral part of the foot are given a stiffer support than is the area corresponding to the big toe and medial front part of the foot.

2. A sole (300) according to claim 1, **characterised in that** a boundary line (302), separating said areas of different flexural resistance, extends from a lateral side of the sole to a medial side of the sole, such that said first area extends essentially over the toe portion and at the same time over a part of the medial side portion of said sole, and that said second area extends over the heel portion and at the same time over most of the lateral side portion of the sole.

3. A sole according to claim 1, **characterised in that** it comprises three or more areas of different flexural resistance (I-IV) being separated by boundary lines (301-303).

4. A sole according to claim 3, **characterised in that** one or more of the boundary lines runs from a lateral side edge (310) of the sole (300) to a medial side edge (320) of the sole.

5. A sole according to claim 3, **characterised in that** a finishing point (331) of a boundary line at said medial side edge (320) of the sole is located clearly further away from the toe end of the sole, than a starting point (341) of said line at the lateral side edge (310) of the sole.

6. A sole according to claim 1, **characterised in that** a boundary line (302), separating said areas of different flexural resistance, has a curved shape.

7. A sole according to claim 6, **characterised in that** said boundary line resembles an "S".

8. A sole according to claim 3, **characterised in that** one or more of the boundary lines (303) of said sole have the shape of a straight line.

9. A sole according to claim 3, **characterised in that** one or more of the boundary lines (301-303) of said sole curve on at least one location (351-354) along the line.

10. A sole according to claim 9, **characterised in that** one or more of the boundary lines of said sole curve towards the toe end at one location (351) and towards the heel end at another location (352).

11. A sole according to claim 3, **characterised in that** one or more of the boundary lines (301-303) of said sole have a shape resembling a hyperbolic tangent graph or a mirrored hyperbolic tangent graph.

12. A sole according to claim 3, **characterised in that** said first area (I) of the sole have lesser flexural resistance, i.e. are more flexible than the rest of the sole (II-IV).

13. A sole according to claim 1, **characterised in that** said sole includes one or more of the following materials; composite material, carbon fibre material, glass fibre material, aramid e.g. KEVLAR® fibre material, plastic material, or metal.

14. A sole according to claim 1, **characterised in that** it includes sandwich layers (401-410).

15. A sole according to claim 14, **characterised in that** different layers (401-410) have different extensions as far as how much of the area of the full sole they are covering.

16. A sole according to claim 14, where said layers (401-410) include layers of alternating aramid fibre and carbon fibre.

17. A sole according to claim 14, where said layers (401-410) include layers of different fibre directions.

18. A sole according to claim 14, where said layers include layers of different types of fabric.

19. A prosthesis incorporating a sole according to any of the above claims.

20. A shoe incorporating a sole according to any of the claims 1-18.

21. An orthosis incorporating a sole according to any of the claims 1-18.

## Patentansprüche

1. Sohle (300) zum Plazieren unter dem Fuß eines Patienten in einem Schuh oder als Teil einer orthopädischen Vorrichtung, Material umfassend, welches eingerichtet ist, um die Sohle mit einer ersten Biegefestigkeit in einem ersten Bereich der Sohle und einer zweiten Biegefestigkeit in einem zweiten Bereich der Sohle auszustatten, **dadurch gekennzeichnet, dass** die ersten und zweiten Bereiche in der Weise eingerichtet sind, dass der kleinen Zehe und einem der Körpermitte abgewandten Teil des Fußes eine festere Stütze gegeben wird als dem Bereich, welcher der großen Zehe und einem der Körpermitte zugewandten vorderen Teil des Fußes entspricht.

2. Sohle (300) nach Anspruch 1, **dadurch gekennzeichnet, dass** sich eine Grenzlinie (302), welche die Bereiche unterschiedlicher Biegefestigkeit trennt, von einer der Körpermitte abgewandten Seite der Sohle zu einer der Körpermitte zugewandten Seite der Sohle erstreckt, so dass sich der erste Bereich im Wesentlichen über den Zehenabschnitt und gleichzeitig über einen Teil des der Körpermitte zugewandten Seitenabschnitts der Sohle erstreckt, und dass sich der zweite Bereich über den Fersenabschnitt und gleichzeitig über den größten Teil des der Körpermitte abgewandten Seitenabschnitts der Sohle erstreckt.

3. Sohle nach Anspruch 1, **dadurch gekennzeichnet, dass** sie drei oder mehr Bereiche unterschiedlicher Biegefestigkeit (1 - IV) umfasst, die durch Grenzlinien (301 - 303) voneinander getrennt sind.

4. Sohle nach Anspruch 3, **dadurch gekennzeichnet, dass** eine oder mehrere der Grenzlinien von einer der Körpermitte abgewandten Seitenkante (310) der Sohle (300) zu einer der Körpermitte zugewandten Seitenkante (320) der Sohle verlaufen.

5. Sohle nach Anspruch 3, **dadurch gekennzeichnet, dass** ein Endpunkt (331) einer Grenzlinie an der der Körpermitte zugewandten Seitenkante (320) der Sohle deutlich weiter entfernt von dem Zehen-Ende der Sohle positioniert ist, als ein Ausgangspunkt (341) der Linie an der der Körpermitte abgewandten Seitenkante (310) der Sohle.

6. Sohle nach Anspruch 1, **dadurch gekennzeichnet, dass** eine Grenzlinie (302), die die Bereiche unterschiedlicher Biegefestigkeit trennt, eine gebogene Form aufweist.

7. Sohle nach Anspruch 6, **dadurch gekennzeichnet, dass** die Grenzlinie einem "S" ähnelt.

8. Sohle nach Anspruch 3, **dadurch gekennzeichnet, dass** eine oder mehrere der Grenzlinien (303) der Sohle die Form einer geraden Linie aufweisen.

9. Sohle nach Anspruch 3, **dadurch gekennzeichnet, dass** eine oder mehrere der Grenzlinien (301 - 303) der Sohle an zumindest einer Position (351 - 354) entlang der Linie gebogen sind.

10. Sohle nach Anspruch 9, **dadurch gekennzeichnet, dass** eine oder mehrere der Grenzlinien der Sohle an einer Position (351) in Richtung des Zehen-Endes und an einer anderen Position (352) in Richtung des Fersen-Endes gebogen sind.

11. Sohle nach Anspruch 3, **dadurch gekennzeichnet, dass** eine oder mehrere der Grenzlinien (301 - 303) der Sohle eine Form aufweisen, die einer Hyperbeltangens-Kurve oder einer gespiegelten Hyperbeltangens-Kurve ähnelt.

12. Sohle nach Anspruch 3, **dadurch gekennzeichnet, dass** der erste Bereich (I) der Sohle eine geringere Biegefestigkeit aufweist, das heißt flexibler ist als der Rest der Sohle (II - IV).

13. Sohle nach Anspruch 1, **dadurch gekennzeichnet, dass** die Sohle eine oder mehr der folgenden Materialien einschließt: Verbundstoff, Kohlefaser, Glasfaser, Aramid z.B. KEVLAR ® - Faser, Kunststoff oder Metall.

14. Sohle nach Anspruch 1, **dadurch gekennzeichnet, dass** sie Sandwich-Lagen (401 - 410) einschließt.

15. Sohle nach Anspruch 14, **dadurch gekennzeichnet, dass** verschiedene Lagen (401 - 410) unterschiedliche Ausdehnungen aufweisen, je nachdem, wieviel sie von dem Bereich der ganzen Sohle bedecken.

16. Sohle nach Anspruch 14, wobei die Lagen (401 - 410) Lagen von sich abwechselnden Aramidfasern und Kohlefasern einschließen.

17. Sohle nach Anspruch 14, wobei die Lagen (401 - 410) Lagen unterschiedlicher Faserrichtungen einschließen.

18. Sohle nach Anspruch 14, wobei die Lagen Lagen von unterschiedlichen Arten von Gewebe einschließen.

19. Prothese, welche eine Sohle nach einem der vorstehenden Ansprüche einschließt.

20. Schuh, welcher eine Sohle nach einem der Ansprüche 1-18 einschließt.

21. Orthese, welche eine Sohle nach einem der Ansprüche 1-18 einschließt.

## Revendications

1. Semelle (300) destinée à être placée sous le pied d'un patient à l'intérieur d'une chaussure ou faisant partie d'un appareil orthopédique, comprenant un matériau agencé pour donner à ladite semelle une première résistance à la flexion au niveau d'une première région de ladite semelle et une seconde résistance à la flexion au niveau d'une seconde région de ladite semelle, **caractérisée en ce que** lesdites première et seconde régions sont agencées de sorte que le petit orteil et la partie latérale du pied donnent un support plus rigide que dans la région correspondant au gros orteil et à la partie avant interne du pied.

2. Semelle (300) selon la revendication 1, **caractérisée en ce qu'**une ligne de délimitation (302), séparant lesdites régions de résistance de flexion différentes, s'étend à partir d'un côté latéral de la semelle jusqu'à un côté interne de la semelle, de sorte que ladite première région s'étend essentiellement sur la partie de pointe et en même temps sur une partie de la partie latérale interne de ladite semelle et de sorte que ladite seconde région s'étend sur la partie de talon et en même temps sur la majeure partie de la partie latérale de la semelle.

3. Semelle selon la revendication 1, **caractérisée en ce qu'**elle comprend trois ou plusieurs régions de résistance à la flexion différentes (I - IV) qui sont séparées par des lignes de délimitation (301 - 303).

4. Semelle selon la revendication 3, **caractérisée en ce qu'**une ou plusieurs des lignes de délimitation s'étend à partir d'un bord latéral (310) de la semelle (300) jusqu'à un bord latéral interne (320) de la semelle.

5. Semelle selon la revendication 3, **caractérisée en ce qu'**un point final (331) d'une ligne de délimitation au niveau dudit bord latéral interne (320) de la semelle est situé clairement plus loin de l'extrémité de pointe de la semelle qu'un point de départ (341) de ladite ligne au niveau du bord latéral (310) de la semelle.

6. Semelle selon la revendication 1, **caractérisé en ce qu'**une ligne de délimitation (302), séparant lesdites régions de résistance de flexion différentes, a une forme incurvée.

7. Semelle selon la revendication 6, **caractérisée en ce que** ladite ligne de délimitation ressemble à un « S ».

8. Semelle selon la revendication 3, **caractérisée en ce qu'**une ou plusieurs des lignes de délimitation (303) de ladite semelle a/ont la forme d'une ligne droite.

9. Semelle selon la revendication 3, **caractérisée en ce qu'**une ou plusieurs des lignes de délimitation (301 - 303) de ladite semelle se courbent sur au moins un emplacement (351 - 354) le long de la ligne.

10. Semelle selon la revendication 9, **caractérisée en ce qu'**une ou plusieurs des lignes de délimitation de ladite semelle se courbent vers l'extrémité de pointe au niveau d'un emplacement (351) et vers l'extrémité de talon au niveau d'un autre emplacement (352).

11. Semelle selon la revendication 3, **caractérisée en ce qu'**une ou plusieurs des lignes de délimitation (301- 303) de ladite semelle ont une forme ressemblant à un graphique de tangente hyperbolique ou à un graphique de tangente hyperbolique en miroir.

12. Semelle selon la revendication 3, **caractérisée en ce que** ladite première région (I) de la semelle a une résistance à la flexion moindre, c'est-à-dire qu'elle est plus flexible que le reste de la semelle (II - IV).

13. Semelle selon la revendication 1, **caractérisée en ce que** ladite semelle comprend un ou plusieurs des matériaux suivants : matériau composite, matériau en fibres de carbone, matériau en fibres de verre, aramide par exemple un matériau en fibres de Kevlar®, matière plastique ou métal.

14. Semelle selon la revendication 1, **caractérisée en ce qu'**elle comprend des couches (401-410) en sandwich.

15. Semelle selon la revendication 14, **caractérisée en ce que** les différentes couches (401 - 410) ont des extensions différentes en fonction de la quantité de surface de toute la semelle qu'elles couvrent.

16. Semelle selon la revendication 14, dans laquelle lesdites couches (401- 410) comprennent des couches de fibres d'aramide et de fibres de carbone alternées.

17. Semelle selon la revendication 14, dans laquelle lesdites couches (401- 410) comprennent des couches de différentes directions de fibres.

18. Semelle selon la revendication 14, dans laquelle lesdites couches comprennent des couches de différents types de tissu.

19. Prothèse comprenant une semelle selon l'une quelconque des revendications ci-dessus.

20. Chaussure comprenant une semelle selon l'une quelconque des revendications 1 à 18.

21. Orthèse comprenant une semelle selon l'une quelconque des revendications 1 à 18.
